# FASCICULE DE BREVET EUROPEEN

(11) **EP 2 326 615 B1**
(45) Date de publication et mention de la délivrance du brevet: **03.09.2014**
(21) Numéro de dépôt: 09747879.6
(22) Date de dépôt: 14.09.2009
(51) Int. Cl.: C07C 57/04, C07C 51/25, C08F 220/06, C07C 51/44

(54) **PROCEDE DE FABRICATION D'ACIDE ACRYLIQUE BIO-RESSOURCE DE GRADE POLYMERE A PARTIR DE GLYCEROL**
VERFAHREN ZUR HERSTELLUNG VON AUS BIOLOGISCHEN QUELLEN STAMMENDER ACRYLSÄURE MIT POLYMERGÜTE AUS GLYCERIN
METHOD FOR PRODUCING BIO-RESOURCED POLYMER-GRADE ACRYLIC ACID FROM GLYCEROL

(30) Priorité: 16.09.2008 FR 0856212
(43) Date de publication de la demande: 01.06.2011
(73) Titulaire: Arkema France, 92700 Colombes (FR)
(72) Inventeur: DEVAUX, Jean-François, 69510 Soucieu en Jarrest (FR); FAUCONET, Michel, 57730 Valmont (FR); LAURENT, Denis, 57500 Saint-avold (FR)
(74) Mandataire: Bonnel, Claudine
(86) Numéro de dépôt international: PCT/FR2009/051718
(87) Numéro de publication internationale: WO 2010/031949

(56) Documents cités:
- EP-A- 0 270 999
- WO-A-2006/092272
- WO-A-2006/136336
- WO-A-2008/087315
- DE-A1- 10 138 150
- US-A- 3 689 541
- US-A- 3 725 208
- US-A- 3 932 500

## Description

La présente invention vise un procédé de fabrication d'un acide acrylique bio-ressourcé de grade polymère à partir de glycérol comme matière première. Le terme bio-ressourcé indique que l'acide acrylique est essentiellement fondé sur une source de carbone d'origine naturelle.

L'acide acrylique est un composé qui est utilisé essentiellement, sinon quasiment uniquement, comme monomère ou co-monomère de polymérisation pour la fabrication d'une très large gamme de produits finaux. Ces produits finaux sont fabriqués par polymérisation de l'acide, des dérivés de cet acide, sous forme ester (polyacrylates) ou amide (polyacrylamides). Un débouché très important de l'acide acrylique est la fabrication de super-absorbants dans laquelle on polymérise un acide acrylique partiellement neutralisé (mélange acide acrylique et acrylate de sodium ou d'autres cations), ou bien on polymérise l'acide acrylique et on neutralise partiellement le composé polyacrylique obtenu. Ces polymères sont utilisés tels quels ou en tant que copolymères dans des domaines aussi variés que l'hygiène, les détergents, les peintures, les vernis, les adhésifs, le papier, les textiles, le cuir, etc...

Les industriels ont développé depuis des décennies des procédés de synthèse de l'acide acrylique.

La première génération utilisait comme matière première des composés à triple liaison, de type acétylénique, que l'on faisait réagir avec un mélange de monoxyde de carbone et d'eau en présence d'un catalyseur à base de nickel.

La deuxième génération de procédés, celle qui aujourd'hui est la plus largement exploitée industriellement met en oeuvre une réaction d'oxydation catalytique du propylène et/ou de propane à l'aide d'oxygène ou d'un mélange contenant de l'oxygène. Cette réaction est conduite généralement en phase gazeuse, et le plus souvent en deux étapes : la première étape réalise l'oxydation sensiblement quantitative du propylène en un mélange riche en acroléine, dans lequel l'acide acrylique est minoritaire puis, lors de la deuxième étape réalise l'oxydation sélective de l'acroléine en acide acrylique.
Les conditions de réaction de ces deux étapes, réalisées dans deux réacteurs en série ou dans un seul réacteur contenant les 2 étages de réaction en série, sont différentes et nécessitent des catalyseurs adaptés à la réaction ; il n'est cependant pas nécessaire d'isoler l'acroléine de première étape au cours de ce procédé en deux étapes.
Les matières premières utilisées sont issues du pétrole ou de gaz naturel, et par conséquent l'acide acrylique est constitué à partir d'une matière première carbonée fossile non renouvelable. En outre, les procédés d'extraction, purification et synthèse des matières premières ainsi que les processus de destruction en fin de cycle des produits finis fabriqués à base de ces matières premières fossiles génèrent du dioxyde de carbone, ce dernier étant un sous-produit direct des réactions d'oxydation du propylène en acroléine puis d'acroléine en acide acrylique. Tout ceci contribue à l'accroissement de la concentration des gaz à effets de serre dans l'atmosphère. Dans le cadre des engagements de la plupart des pays industrialisés à réduire les émissions de gaz à effet de serre, il apparaît particulièrement important de fabriquer de nouveaux produits à base de matière première renouvelable contribuant à réduire ces effets environnementaux.

Depuis quelques années, les industriels conduisent des travaux de recherche et de développement sur des procédés de synthèse dits « bio-ressourcés » utilisant des matières premières naturelles renouvelables. En effet, pour limiter l'impact écologique des procédés de production classiques, des procédés alternatifs à partir de matières premières végétales non fossiles ont été développés récemment. Il s'agit par exemple de procédés utilisant comme matière première l'acide 2-hydroxypropionique (acide lactique), obtenu par fermentation de glucose ou de mélasse provenant de la biomasse. Il s'agit également de procédés à partir de glycérol (appelé aussi glycérine), issu de la méthanolyse des huiles végétales en même temps que les esters méthyliques qui sont, eux, employés notamment comme carburants ou combustibles dans le gazole et le fioul domestique. La méthanolyse des huiles végétales ou des graisses animales peut s'effectuer selon différents procédés bien connus, notamment en utilisant une catalyse homogène telle que la soude ou le méthylate de sodium en solution dans le méthanol, ou en utilisant une catalyse hétérogène. On pourra se reporter sur ce sujet à l'article de D. Ballerini et al. dans l'Actualité Chimique de nov-déc 2002.
Les procédés utilisant l'acide hydroxypropionique comme matière première ont un inconvénient majeur du point de vue économique. Ils mettent en jeu une réaction de fermentation qui est réalisée nécessairement en condition très diluée dans l'eau. Pour obtenir l'acide acrylique, une quantité très importante d'eau doit être éliminée par distillation, au prix d'un coût énergétique très important. Par ailleurs, l'énergie dépensée pour séparer l'eau, énergie produite à partir de matière fossile, vient dégrader fortement l'avantage initial de produire l'acide acrylique à partir de cette matière première bio-ressourcée. On peut citer dans ce domaine la demande WO2006/092271 qui décrit un procédé de production de polymères à partir d'acide acrylique préparé par voie enzymatique, notamment à partir de carbohydrate.

Il est connu depuis longtemps qu'il est possible à partir de matériaux organiques naturels tels que les polyols susceptibles d'être transformés par voie chimique pour obtenir des acides ou des aldéhydes comportant 3 atomes de carbone par molécule qui peuvent constituer des précurseurs de l'acide acrylique. A titre d'exemple on peut citer la synthèse de l'acroléine obtenue par déshydratation de glycérol qui est notamment décrite dans le brevet US 5,387,720. Le glycérol (appelé aussi glycérine) est issu de la méthanolyse des huiles végétales en même temps que les esters méthyliques qui sont, eux, employés notamment comme carburants ou combustibles dans le gazole et le fioul domestique. C'est un produit naturel qui jouit d'une aura "verte", il est disponible en grande quantité et peut être stocké et transporté sans difficulté. De nombreuses études sont consacrées à la valorisation du glycérol selon son degré de pureté, et la déshydratation du glycérol en acroléine est une des voies envisagées.

La réaction mise en jeu pour obtenir l'acroléine à partir du glycérol est :

CH₂OH-CHOH-CH₂PH → CH₂=CH-CHO + 2H₂O

Cette étape est suivie d'une étape d'oxydation classique de l'acroléine pour obtenir l'acide acrylique selon la réaction :

CH₂=CH-CHO + ½ O₂ → CH₂=CH-COOH

Les demandes de brevet EP1.710.227, WO2006/135336, WO2006/092272 et WO2007/0219521 décrivent des procédés de synthèse d'acide acrylique à partir de glycérol comportant l'étape de déshydratation en phase gaz en présence de catalyseurs constitués par des oxydes minéraux (mixtes ou non) à base d'aluminium, titane, zirconium, vanadium, etc., et l'étape d'oxydation en phase gaz de l'acroléine ainsi synthétisé en présence de catalyseurs à base d'oxydes de fer, molybdène, cuivre etc., seuls ou en combinaison sous forme d'oxydes mixtes.

Cependant ils ne donnent pas de détails précis sur la phase de purification de l'acide acrylique et ne décrivent pas du tout quelles impuretés contient ou ne contient pas l'acide acrylique obtenu. Par exemple, la demande WO2006/092272 décrit un procédé de fabrication d'acide acrylique et de superabsorbant à partir de glycérol. Il y est affirmé que l'on peut obtenir un acide acrylique de pureté 99 à 99,98% sans préciser comment il est obtenu et quelles sont les impuretés résiduelles. Dans l'exemple 2, il est précisé simplement que l'on obtient un acide acrylique qui ne contient pas de protoanémonine sans considérer les autres impuretés susceptibles d'avoir une incidence sur les polymérisations ultérieures.

L'acide acrylique est destiné à la mise en oeuvre par les industriels de procédés de polymérisation soit de l'acide acrylique, soit de ses dérivés esters, procédés qui sont conduits sous différentes formes en masse, en solution, en suspension, en émulsion. Ces procédés peuvent être très sensibles à la présence dans la charge de certaines impuretés, comme les aldéhydes ou les composés insaturés, qui peuvent parfois empêcher d'obtenir la valeur d'usage attendue, par exemple en limitant la conversion du monomère en polymère, en limitant la longueur de chaîne du polymère ou en interférant dans la polymérisation dans le cas de composés insaturés. D'autres impuretés, comme les composés saturés non polymérisables, peuvent être particulièrement gênants dans l'application finale, en modifiant les propriétés du produit fini, en conférant des propriétés toxiques ou corrosives au produit fini, ou encore en augmentant les rejets organiques polluants lors des étapes de fabrication du polymère et/ou du produit fini.

Les exploitants se montrent exigeants en matière de spécifications de qualité de l'acide acrylique (ou de son ester). Celui-ci doit répondre à des seuils sévères en matière d'impuretés. En effet, les utilisateurs d'acide acrylique ou d'esters acryliques qui produisent des polymères mettent en oeuvre des recettes adaptées pour la production de leurs polymères à partir d'une qualité « standard » d'acide acrylique ou d'esters fabriquée aujourd'hui uniquement à partir de propylène. Une modification des recettes utilisées par ces utilisateurs, en vue des les adapter à une qualité différente d'acide acrylique ou d'esters produits par une autre voie que celle des procédés conventionnels ex-propylène présenterait des inconvénients importants pour ces sociétés utilisatrices. Hormis des coûts de recherche et développement supplémentaires, la production d'un type de polymères sur une même unité à partir de qualités différentes d'acide acrylique ou d'esters selon leur origine, fossiles ou bio-ressourcées (comme le glycérol), occasionnerait des coûts d'adaptation importants et une infrastructure de production plus compliquée.

Le besoin se fait maintenant sentir de la mise sur le marché d'un acide acrylique qui réponde à l'ensemble des contraintes évoquées ci-dessus aussi bien en amont, c'est-à-dire un acide acrylique essentiellement fondé sur une source de carbone naturelle non fossile, qu'en aval c'est-à-dire un acide acrylique répondant à des normes de qualité permettant son exploitation dans la fabrication d'une gamme étendue de polymères techniques, sans toutefois nécessiter une purification sophistiquée et donc coûteuse.

La présente invention a pour but de pallier les inconvénients antérieurs en proposant un procédé de fabrication d'un acide acrylique bio-ressourcé de grade polymère, ce grade étant défini par les seuils limites en teneur des impuretés préjudiciables à une large gamme de procédés de polymérisation.

L'invention a pour objet un procédé de fabrication d'un acide acrylique bio-ressourcé de grade polymère ayant une teneur poids en acide acrylique > 99 % et les teneurs en impuretés suivantes :
aldéhydes totaux < 10 ppm
protoanémonine < 5 ppm
anhydride maléique < 30 ppm
inhibiteurs de polymérisation non phénoliques < 10 ppm
et une teneur en masse de ¹⁴C telle que le ratio ¹⁴C /¹²C > 0,8.10⁻¹².

L'acide acrylique obtenu par le procédé de l'invention aura de préférence une teneur en
protoanémonine < 3 ppm
aldéhydes totaux < 3 ppm
anhydride maléique < 15 ppm
inhibiteurs de polymérisation non phénoliques < 3 ppm et une teneur en masse de ¹⁴C telle que le ratio ¹⁴C/¹²C > 1.10⁻¹².

L'invention vise un procédé de fabrication d'un acide acrylique de grade polymère en utilisant le glycérol comme matière première qui sera transformé en deux étapes - déshydratation et oxydation - telles que mentionnées précédemment intégrées dans un processus global de purification.
Ce procédé présente avec le procédé de synthèse à partir du propylène une grande analogie dans la mesure où le produit intermédiaire, l'acroléine, issu de la première étape est le même et que la seconde étape est conduite dans les mêmes conditions opératoires. Cependant, la réaction de première étape du procédé de l'invention, réaction de déshydratation, est différente de la réaction d'oxydation du propylène du procédé habituel. La réaction de déshydratation menée en phase gaz est conduite au moyen de catalyseurs solides différents de ceux utilisés pour l'oxydation du propylène. L'effluent riche en acroléine issu de la première étape de déshydratation, destiné à alimenter la seconde étape d'oxydation de l'acroléine en acide acrylique, contient une quantité plus importante d'eau, et présente en outre des différences sensibles en matière de sous-produits découlant des mécanismes réactionnels mis en jeu se concrétisant par des sélectivités différentes dans chacune des deux voies.

L'utilisation de matières premières carbonées d'origine naturelle et renouvelable peut se détecter grâce aux atomes de carbone entrant dans la composition du produit final. En effet, à la différence des matériaux issus de matières fossiles, les matériaux composés de matières premières renouvelables contiennent du ¹⁴C. Tous les échantillons de carbone tirés d'organismes vivants (animaux ou végétaux) sont en fait un mélange de 3 isotopes : ¹²C (représentant - 98,892 %), ¹³C (∼ 1,108 %) et ¹⁴C (traces: 1,2.10⁻¹⁰ %). Le rapport ¹⁴C /¹²C des tissus vivants est identique à celui de l'atmosphère. Dans l'environnement, le ¹⁴C existe sous deux formes prépondérantes : sous forme minérale c'est-à-dire de gaz carbonique (CO₂) et sous forme organique, c'est-à-dire de carbone intégré dans des molécules organiques.
Dans un organisme vivant, le rapport ¹⁴C /¹²C est maintenu constant par le métabolisme car le carbone est continuellement échangé avec l'environnement. La proportion de ¹⁴C étant sensiblement constante dans l'atmosphère, il en est de même dans l'organisme, tant qu'il est vivant, puisqu'il absorbe ce ¹⁴C comme il absorbe le ¹²C. Le rapport moyen de ¹⁴C /¹²C est égal à 1,2x10⁻¹².
Le ¹²C est stable, c'est-à-dire que le nombre d'atomes de ¹²C dans un échantillon donné est constant au cours du temps. Le ¹⁴C, lui, est radioactif et chaque gramme de carbone d'être vivant contient suffisamment d'isotope ¹⁴C pour donner 13,6 désintégrations par minute.
La demi-vie (ou période) T_{1/2}, liée à la constante de désintégration du ¹⁴C est de 5730 ans. Compte tenu de cette durée, on considère que la teneur en ¹⁴C est pratiquement constante depuis l'extraction des matières premières végétales jusqu'à la fabrication du produit final..

L'acide acrylique bio-ressourcé de l'invention a une teneur en masse de ¹⁴C telle que le ratio ¹⁴C /¹²C est supérieur à 0,8.10⁻¹², et de préférence supérieur à 1.10⁻¹². Cet acide acrylique bio-ressourcé pourra même atteindre un ratio égal à 1,2.10⁻¹² dans le cas où l'ensemble des éléments carbonés utilisés pour sa fabrication sera d'origine naturelle non fossile.

A l'heure actuelle, il existe au moins deux techniques différentes pour la mesure de la teneur en ¹⁴C d'un échantillon :
- par spectrométrie à scintillation liquide
- par spectrométrie de masse : l'échantillon est réduit en graphite ou en CO₂ gazeux, analysé dans un spectromètre de masse. Cette technique utilise un accélérateur et un spectromètre de masse pour séparer les ions ¹⁴C des ¹²C et donc déterminer le rapport des deux isotopes.

Toutes ces méthodes de mesure de la teneur en ¹⁴C des matériaux sont décrites précisément dans les normes ASTM D 6866 (notamment D6866-06) et dans les normes ASTMD 7026 (notamment 7026-04). La méthode de mesure préférentiellement utilisée est la spectrométrie de masse décrite dans la norme ASTM D6866-06 (« accelerator mass spectroscopy »).

L'invention a pour objet un procédé de fabrication de l'acide acrylique bio-ressourcé de grade polymère à partir de glycérol, comportant les étapes suivantes :
i) déshydratation du glycérol en acroléine,
ii) oxydation de l'acroléine formée en acide acrylique,
iii) extraction de l'acide acrylique formé par absorption à contre-courant sous forme d'une solution aqueuse d'acide acrylique,
iv) déshydratation de la solution en présence d'un solvant de l'acide acrylique non miscible à l'eau mais susceptible de former avec l'eau un azéotrope,
v) élimination des composés légers, en particulier l'acide acétique et l'acide formique, par distillation,
vi) élimination des impuretés lourdes par distillation, pour obtenir une qualité d'acide acrylique de grade "technique",
vii) purification par distillation de l'acide acrylique technique pour obtenir une qualité d'acide acrylique de grade "polymère", après addition à l'acide acrylique d'un composé aminé réagissant avec les aldéhydes encore présents, choisi parmi les dérivés de l'hydrazine, de préférence l'hydrate d'hydrazine

Le glycérol est un produit chimique, le 1,2,3-propane triol, qui peut être obtenu soit par synthèse chimique à partir de propylène, soit comme co-produit formé lors de la méthanolyse des huiles végétales ou des graisses animales. La méthanolyse des huiles végétales qui constitue une étape préliminaire du procédé en cas d'intégration de l'ensemble de la chaîne huile/graisse → acide acrylique, conduit, d'une part à des esters méthyliques, d'autre part à du glycérol. Les esters méthyliques sont employés notamment comme carburants ou combustibles dans le gazole et le fioul domestique. Avec le développement des carburants d'origines renouvelables, et notamment des esters méthyliques d'huiles végétales (EMHV), la production de glycérol selon cette voie d'obtention augmente fortement, le glycérol représentant de l'ordre de 10 % du poids de l'huile transformée.

La glycérine, dénomination du glycérol lorsqu'il est en solution aqueuse, obtenue à partir des huiles végétales ou des graisses animales peut contenir des sels (NaCl, Na₂SO₄, KCl, K₂SO₄...). Dans ce cas, on aura généralement une étape préliminaire d'élimination des sels par exemple par distillation, par utilisation de résines échangeuses d'ions ou par utilisation d'un lit fluidisé tel que décrit dans la demande française FR 2 913 974. Parmi les méthodes utilisées ou étudiées pour la purification et l'évaporation du glycérol, on citera notamment celles qui sont décrites par G.B. D'Souza, dans J. Am. Oil Chemists' Soc. November 1979 (Vol 56) 812A, par Steinberner U et al., dans Fat. Sci. Technol. (1987), 89 Jahrgang Nr.8, pp 297-303, et par Anderson D.D. et al. dans Soaps and Detergents : A theoretical and Practical Review, Miami Beach Fla., Oct 12-14 1994, chapter 6 pp172-206. Ed: L Spitz, AOCS Press, Champaign.

On utilise généralement des solutions aqueuses de glycérol dont la concentration peut varier dans de larges mesures, par exemple de 20 à 99% en poids de glycérol, de préférence on utilise des solutions comportant de 30 à 80% en poids de glycérol.

Le principe du procédé d'obtention d'acide acrylique à partir de glycérol est basé sur les 2 réactions consécutives de déshydratation et d'oxydation :

CH₂OH-CHOH-CH₂OH ↔ CH₂=CH-CHO+2H₂O

CH₂=CH-CHO + ½ O₂ → CH₂=CH-COOH

Le procédé peut être mis en oeuvre en deux étapes distinctes avec deux catalyseurs différents.

La réaction de déshydratation, qui est une réaction équilibrée mais favorisée par un niveau de température élevée, est effectuée en général en phase gaz dans le réacteur en présence d'un catalyseur à une température allant de 150°C à 500°C, de préférence comprise entre 250°C et 350°C, et une pression comprise entre 1 et 5 bars. Elle peut également être conduite en phase liquide. On peut aussi l'effectuer en présence d'oxygène ou d'un gaz contenant de l'oxygène comme décrit dans les demandes WO 06/087083 et WO 06/114506.

La réaction d'oxydation s'effectue en présence d'oxygène moléculaire ou d'un mélange contenant de l'oxygène moléculaire, à une température allant de 200°C à 350°C, de préférence de 250°C à 320°C, et sous une pression allant de 1 à 5 bars en présence d'un catalyseur d'oxydation.

La réaction de déshydratation du glycérol est généralement effectuée, sur des catalyseurs solides acides. Les catalyseurs qui conviennent sont des matériaux homogènes ou multiphases, insolubles dans le milieu réactionnel qui ont une acidité de Hammett, notée Ho inférieure à +2. Comme indiqué dans le brevet US 5,387,720 qui fait référence à l'article de K. Tanabe et al dans "Studies in Surface Science and Catalysis", Vol 51, 1989, chap 1 et 2, l'acidité de Hammett est déterminée par titration amine à l'aide d'indicateurs ou par adsorption d'une base en phase gazeuse.

Ces catalyseurs peuvent être choisis parmi des matériaux siliceux naturels ou de synthèse ou les zéolithes acides ; des supports minéraux, tels que des oxydes, recouverts par des acides inorganiques, mono, di, tri ou polyacides ; des oxydes ou oxydes mixtes ou encore des hétéropolyacides ou sels d'hétéropolyacides.

Ces catalyseurs pourront généralement être constitués par un sel d'hétéropolyacide dans lequel des protons dudit hétéropolyacide sont échangés avec au moins un cation choisi parmi les éléments appartenant aux Groupes 1 à XVI de la Classification Périodique des Eléments, ces sels d'hétéropolyacide contenant au moins un élément choisi parmi le groupe comprenant W, Mo et V.

Parmi les oxydes mixtes, on peut citer particulièrement ceux à base de fer et de phosphore et ceux à base de césium, phosphore et tungstène.

Les catalyseurs sont notamment choisis parmi les zéolithes, les composites Nafion® (à base d'acide sulfonique de polymères fluorés), les alumines chlorées, les acides et sels d'acides phosphotungstiques et/ou silicotungstiques, et différents solides de type oxydes métalliques tels que oxyde de tantale Ta₂O₅, oxyde de niobium Nb₂O₅, alumine Al₂O₃, oxyde de titane TiO₂, zircone ZrO₂, oxyde d'étain SnO₂, silice SiO₂ ou silico-aluminate SiO₂-Al₂O₂, imprégnés de fonctions acides telles que borate BO₃, sulfate SO₄, tungstate WO₃, phosphate PO₄, silicate SiO₂, ou molybdate MoO_{3'} ou un mélange de ces composés.

Les catalyseurs précédents peuvent comprendre en plus un promoteur tel que Au, Ag, Cu, Pt, Rh, Pd, Ru, Sm, Ce, Yt, Sc, La, Zn, Mg, Fe, Co, Ni, or montmorillonite.

Les catalyseurs préférés sont les zircones phosphatées, les zircones tungstées, les zircones silicées, les oxydes de titane ou d'étain imprégnés de tungstate ou phosphotungstate, les alumines ou silices phosphatées, les hétéropolyacides ou sels d'hétéropolyacides, les phosphates de fer et les phosphates de fer comprenant un promoteur.

Comme catalyseur d'oxydation, on utilise tous types de catalyseurs bien connus de l'homme de l'art pour cette réaction. Généralement sont utilisés des solides contenant au moins un élément choisi dans la liste Mo, V, W, Re, Cr, Mn, Fe, Co, Ni, Cu, Zn, Sn, Te, Sb, Bi, Pt, Pd, Ru, Rh, présent sous la forme métallique ou sous forme d'oxyde, de sulfate ou de phosphate. En particulier, sont utilisées les formulations contenant Mo et/ou V et/ou W et/ou Cu et/ou Sb et/ou Fe comme constituants principaux.

Lorsque le procédé est conduit en deux étapes avec deux réacteurs différents, il pourra être avantageux de procéder entre les deux réacteurs à une condensation intermédiaire de l'eau telle que décrite dans la demande WO 08/087315.

La transformation du glycérol en acide acrylique qui est basée sur les 2 réactions consécutives de déshydratation et d'oxydation citées ci-dessus peut également être conduite dans un seul et même réacteur. Dans ce schéma réactionnel, appelé oxydéshydratation, le caractère exothermique de la réaction d'oxydation est compensé par le caractère endothermique de la réaction de déshydratation, ce qui contribue à un meilleur équilibre thermique du procédé. Dans ce type de procédés, on peut soit utiliser deux lits catalytiques différents, déshydratation en amont et oxydation en aval, avec chacun leur catalyseur spécifique, soit un seul lit avec un catalyseur « mixte » constitué par un mélange des catalyseurs de déshydratation et d'oxydation. Cette version du procédé à réacteur unique implique le recyclage (après séparation) de l'acroléine non convertie pour réaliser la phase d'oxydation.

Le mélange gazeux issu de la 2ème étape (réaction d'oxydation) est constitué, en dehors de l'acide acrylique :
- de composés légers incondensables dans les conditions de températures et de pression habituellement mises en oeuvre : azote, oxygène non converti, monoxyde et dioxyde de carbone formés en faible quantité par oxydation ultime ou tournant en rond, par recyclage, dans le procédé,
- de composés légers condensables : en particulier l'eau, générée par la réaction de déshydratation ou présente comme diluant, l'acroléine non converti, des aldéhydes légers, comme le formaldéhyde et l'acétaldéhyde, l'acide formique et l'acide acétique,
- de composés lourds : furfuraldéhyde, benzaldéhyde, acide et anhydride maléique, acide benzoïque, acide 2-butenoïque, phénol, protoanémonine...

Le second stade de la fabrication consiste à récupérer l'acide acrylique contenu dans l'effluent gazeux issu de la réaction d'oxydation pour le transformer en acide acrylique de grade polymère de l'invention.

La première étape de cette phase de purification (étape iii du procédé selon l'invention) consiste en une extraction de l'acide acrylique par absorption à contre-courant. Pour cela, on introduit le gaz issu du réacteur en pied d'une colonne d'absorption où il rencontre à contre-courant un solvant introduit en tête de colonne. Les composés légers dans les conditions de température et de pression habituellement mises en oeuvre (respectivement plus de 50°C et moins de 2.10⁵ Pa) sont éliminés en tête de cette colonne d'absorption. Le solvant mis en oeuvre dans cette colonne est l'eau. L'eau utilisée comme solvant absorbant peut être amenée par une source extérieure au procédé, mais sera constituée préférentiellement pour partie ou totalement par l'eau produite lors de l'étape de déshydratation et condensée par refroidissement du flux gazeux réactionnel.
Les conditions opérationnelles de cette étape d'absorption sont les suivantes :
Le mélange réactionnel gazeux est introduit en pied de colonne à une température comprise entre 130°C et 250°C. L'eau est introduite en tête de colonne à une température comprise entre 10°C et 60°C. Les quantités respectives d'eau et de mélange réactionnel gazeux sont telles que le ratio massique eau/acide acrylique est compris entre 1/1 et 1/4. L'opération est conduite à la pression atmosphérique.

Dans une variante du procédé, cette colonne d'absorption peut être couplée avec une colonne de distillation des composés très légers, essentiellement l'acroléine non convertie à l'issue de la réaction, présente en faible concentration dans la solution aqueuse d'acide acrylique récupérée en pied de colonne d'absorption. Cette colonne de distillation, fonctionnant sous une pression de 6.10³ à 7.10⁴ Pa, est alimentée en tête par le flux de pied de colonne d'absorption précédente, et permet d'éliminer en tête un flux d'acide acrylique enrichi en acroléine, qui est recyclé en partie inférieure de la colonne d'absorption, pour une élimination finale en tête de cette même colonne. On obtient ainsi un mélange aqueux d'acide acrylique dans l'eau (ratio massique 1/1 à 4/1) débarrassé de l'essentiel de l'acroléine non convertie, que l'on nomme "acide acrylique brut".

La deuxième étape de cette phase de purification (étape iv) est une étape de déshydratation qui est réalisée en présence d'un solvant de l'acide acrylique non miscible à l'eau, mais susceptible de former avec l'eau un azéotrope.
Ce solvant sera par exemple choisi parmi les solvants suivants : méthylisobutylcétone (MIBK), triméthylcyclohexanone, acétate de butyle, acétate d'isobutyle, acétate d'éthyle, butanoate d'éthyle, heptane, naphtalène, diisobutylène, méthacrylate d'éthyle, acrylate ou méthacrylate de propyle, toluène, mélange toluène + alcools en C₄-C₈, mélange d'un solvant 1 (acétate de propyle ou acétate d'éthyle ou méthacrylate d'éthyle ou acrylate de propyle ou propionate de propyle) + un solvant 2 (alcool en C₄-C₈), mélange d'un solvant 1 (hydrocarbure en C₇ ou toluène) + un solvant 2 (ester : acétate d'éthyle, acrylate de méthyle, acrylate de propyle ou acrylate d'éthyle, ou nitrile : acétonitrile ou acrylonitrile), mélange d'un solvant 1 (diéthylcétone ou méthylpropylcétone ou MIBK ou acétate de propyle) + un solvant 2 (toluène, heptane ou méthylcyclohexane)...

Dans une première forme de réalisation, cette étape de déshydratation peut être réalisée par une extraction liquide - liquide de l'acide acrylique en présence du solvant, suivie par une étape de séparation du monomère, acide acrylique, par distillation de la phase organique.

Cette phase de déshydratation est décrite dans de nombreux brevets, voir par exemple le brevet FR 2.119.764, avec la méthylisobutylcétone (MIBK) comme solvant, ou le brevet US 3,689,541, avec la triméthylcyclohexanone comme solvant, ou par distillation en présence de solvant ou de mélanges de solvants formant un azéotrope hétérogène avec l'eau, comme les acétates ou la méthylisobutylcétone tel que décrit par exemple dans le brevet FR 2.554.809 ou encore des solvants formant en outre un mélange azéotropique avec l'acide acétique, comme le toluène tel que décrit par exemple dans le brevet JP 03.181.440.

Dans une seconde forme de réalisation, on utilisera dans le procédé de l'invention de préférence pour cette étape de déshydratation, une distillation azéotropique de la solution aqueuse d'acide acrylique à l'aide d'un des solvants cités ci-dessus et notamment en présence d'un solvant tel que MIBK. La colonne de distillation qui fonctionne sous une pression de 6.10³ à 7.10⁴ Pa, est équipée d'un décanteur qui reçoit le flux de tête de colonne après condensation et assure la séparation d'une phase organique supérieure essentiellement constituée du solvant, totalement recyclée en reflux en tête de colonne, et d'une phase aqueuse contenant l'eau et la majeure partie du formaldéhyde. La puissance de chauffe imposée au bouilleur de la colonne est régulée de façon à obtenir un débit de reflux de solvant tel que le ratio massique de solvant renvoyé en reflux et de l'eau contenue dans l'acide acrylique brut alimentant la colonne corresponde au mélange azéotropique théorique (par exemple : 3/1 dans le cas du solvant MIBK). Le flux obtenu en pied de colonne, l'acide acrylique déshydraté, est essentiellement exempt d'eau (généralement moins de 1 % poids).

Dans cette variante de réalisation, cette colonne peut être couplée à une deuxième colonne de récupération du solvant, de façon à récupérer dans le flux aqueux décanté en tête de colonne de distillation azéotropique les traces de solvant solubilisées dans la phase aqueuse. Ces faibles quantités de solvant distillées et condensées en tête de cette colonne de récupération de solvant, fonctionnant sous pression atmosphérique, sont ensuite recyclées dans le décanteur de la colonne précédente. Le flux aqueux de pied de cette colonne de récupération de solvant est éliminé.

La troisième étape de cette phase de purification (étape v) est une étape d'élimination des légers, en particulier l'acide acétique et l'acide formique, par distillation ; elle est généralement dénommée « étêtage ». Le flux d'acide acrylique déshydraté obtenu en pied de la colonne de distillation azéotropique est envoyé en partie médiane d'une colonne à distiller qui fonctionne sous une pression de tête de l'ordre de 2.10³ à 2.10⁴ Pa. Le flux de pied de colonne contient l'acide acrylique débarrassé de l'essentiel des composés légers. Le flux de tête de colonne, riche en acide acétique et acide formique, peut éventuellement être traité complémentairement pour récupérer dans une deuxième colonne en série les faibles quantités d'acide acrylique entraînées avec le flux de tête de colonne.

La quatrième étape de cette phase de purification (étape vi) est une étape de séparation des composés lourds par distillation. Le flux de pied de la colonne précédente d'étêtage est introduit en pied d'une colonne de distillation fonctionnant sous une pression de tête de l'ordre de 2.10³ à 2.10⁴ Pa. On obtient en tête un flux d'acide acrylique purifié, de grade technique.

La cinquième étape de cette phase de purification (étape vii) consiste à purifier finalement l'acide acrylique de qualité technique en un grade polymère d'acide acrylique. Pour atteindre cette qualité d'acide acrylique permettant de synthétiser des polymères de haute masse moléculaire, il est particulièrement important d'éliminer certains aldéhydes, comme le furfuraldéhyde, le benzaldéhyde et l'acroléine, jusqu'à des teneurs extrêmement faibles, qui ne peuvent être atteintes de manière économique par une simple distillation, en raison de leur volatilité trop proche de celle de l'acide acrylique. Pour ce faire, on peut réaliser une élimination des aldéhydes par un traitement chimique à l'aide d'un réactif qui forme avec ces aldéhydes des produits de réaction lourds plus facilement séparables de l'acide acrylique par distillation. Parmi les réactifs utilisables, on peut employer des amines, comme décrit dans le brevet US 3.725.208, et plus particulièrement les composés de la famille des hydrazines, tel que la glycine comme décrit dans le brevet JP 7.500.014 ou l'hydrate d'hydrazine comme décrit dans les brevets US 3.725.208 ou JP 7.430.312, ou encore l'aminoguanidine comme décrit dans le brevet EP 270.999. Ces composés peuvent être utilisés tels quels ou sous forme de leurs sels. Les traitements chimiques qui sont décrits présentent tous l'inconvénient de générer de l'eau lors de la réaction de l'aldéhyde avec le réactif aminé. La présence de cette impureté dans l'acide acrylique peut également être dommageable pour la fabrication de certains polymères. Pour cette raison, il peut être intéressant de réaliser cette opération de traitement chimique pendant une étape de distillation visant à éliminer l'eau et les composés légers en tête, avant une étape de distillation de l'acide acrylique destinée à séparer les composés lourds, comme il est décrit dans le brevet JP 7.495.920.

Dans un mode de réalisation préféré du procédé de l'invention, le flux d'acide acrylique technique est envoyé en alimentation d'une colonne de distillation fonctionnant sous une pression de tête de l'ordre de 2.10³ à 2.10⁴ Pa, en mélange avec un dérivé de l'hydrazine, de préférence l'hydrate d'hydrazine, introduit dans un ratio molaire de 2 à 10 par rapport aux aldéhydes contenus dans l'acide acrylique technique. Le flux de tête de colonne, composé essentiellement d'acide acrylique, d'eau, et d'acide acétique en faible concentration, peut être recyclé en amont du procédé, par exemple dans le flux d'acide acrylique brut ou en alimentation de la colonne d'étêtage, afin de récupérer l'acide acrylique. Le flux de pied de colonne est quant à lui envoyé en pied d'une deuxième colonne fonctionnant sous une pression de tête de l'ordre de 2.10³ à 2.10⁴ Pa, dans laquelle on réalise l'élimination des composés lourds en fond et la distillation de l'acide acrylique en tête, pour obtenir une qualité "grade polymère" d'acide acrylique.

L'acide acrylique est un produit facilement polymérisable, en particulier pendant les étapes de la purification des flux riches en ce monomère, où les conditions de température relativement élevées sont favorables à l'initiation de la polymérisation. Le polymère d'acide acrylique étant insoluble dans le monomère, il est indispensable d'introduire des inhibiteurs de polymérisation dans les flux riches en acide acrylique, au niveau de tous les équipements du procédé de purification.

Les inhibiteurs de polymérisation généralement utilisés pour les étapes de purification de l'acide acrylique sont les produits phénoliques, comme l'hydroquinone ou l'éther méthylique de l'hydroquinone, les dérivés de la phénothiazine, des composés de la famille des thiocarbamates, comme le di n-butyl dithiocarbamate de cuivre, des dérivés aminés, comme les hydroxylamines, l'hydroxydiphénylamine, ou de la famille des phénylène diamines, des dérivés nitroxydes de la 4-hydroxy 2,2,6,6-tetraméthyl-1-oxyl-pipéridine (TEMPO), comme le 4-hydroxy-TEMPO ou le 4-oxo-TEMPO, ou encore des sels métalliques, comme l'acétate de manganèse. Ces inhibiteurs peuvent être utilisés seuls ou en combinaison, et sont en outre préférentiellement introduits en association avec un gaz contenant de l'oxygène.

Ces inhibiteurs de polymérisation sont généralement des composés lourds, dont la volatilité est plus faible que celle de l'acide acrylique. Ils sont éliminés en fond des colonnes. En revanche, leur concentration dans la phase vapeur à l'intérieur des colonnes de distillation est faible et insuffisante pour éviter l'initiation de polymères. Pour éviter l'apparition et l'accumulation de polymères, ces additifs sont habituellement introduits dans les flux liquides alimentant les équipements, mais aussi en tête et en différents points des colonnes et équipements, de façon à assurer un reflux constant et homogène de solution riche en inhibiteurs de polymérisation sur toutes les parties des équipements. En général, ils sont envoyés en solution dans un liquide, par exemple dans l'acide acrylique ou dans l'eau si l'étape de purification concerne des flux aqueux.

L'acide acrylique de grade polymère obtenu en tête de la dernière colonne de distillation (étape vii) est additionné d'éther méthylique de l'hydroquinone (EMHQ), à une concentration de 200 +/- 20 ppm ; par addition de cet inhibiteur dans le flux de tête de colonne, on stabilise le produit final.

L'acide acrylique bio-ressourcé de grade polymère obtenu selon le procédé de l'invention, est utilisé pour la fabrication de superabsorbants comprenant la polymérisation dudit acide partiellement neutralisé, ou la polymérisation dudit acide suivie d'une neutralisation partielle de l'acide polyacrylique obtenu.

L'acide acrylique bio-ressourcé de grade polymère obtenu selon le procédé de l'invention, est utilisé pour la fabrication de polymères ou de copolymères par polymérisation des dérivés dudit acide sous forme ester ou amide.

L'acide acrylique de l'invention et son procédé de fabrication sont illustrés dans les exemples suivants :
Exemple 1 : étape préliminaire de purification du glycérol brut.
Exemple 2 : transformation du glycérol en acroléine suivie d'une condensation de l'eau
Exemple 3 : oxydation de l'acroléine en acide acrylique.
Exemple 4 : récupération de l'acide acrylique brut sous forme de solution aqueuse.
Exemple 5 : purification de l'acide acrylique brut pour obtenir l'acide acrylique technique.
Exemple 6 : purification de l'acide acrylique technique pour obtenir l'acide acrylique grade polymère.
Exemple 7 : comparatif.
Dans les exemples qui suivent, les concentrations des très faibles d'impuretés dans les flux d'acide acrylique technique et d'acide acrylique de grade polymère sont mesurées par les méthodes suivantes (chiffres entre parenthèses : précision ; seuil de quantification) :
- par chromatographie en phase liquide haute performance sur colonne Lichrospher 100-RP-18, avec détection par spectrométrie UV et quantification par étalonnage externe : protoanémonine (3%; 0,1ppm), furfural (1,4%; 0,1ppm), benzaldéhyde (0,2%; 0,2ppm), anhydride maléique dosé sous forme d'acide maléique (1,5%; 0,1ppm), phénothiazine (2%; 0,2ppm)
- par la même méthode, avec dérivatisation préalable en présence de dinitrophényl hydrazine : formaldéhyde (3%; 0,1 ppm).
- par spectrométrie UV-visible, après réaction de l'acroléine avec le 4-hexyl resorcinol en milieu éthanol/acide trichloracétique, catalysée par le chlorure mercurique, et développement d'une coloration bleue présentant une absorbance maximale à 603nm : acroléine (5%; 0,1ppm)
- par chromatographie en phase gazeuse sur colonne FFAP, avec détection par ionisation de flamme et quantification par étalonnage interne : acide acétique (3%; 10ppm)

### Exemple 1

La première phase consiste à purifier le glycérol brut obtenu à partir de la méthanolyse d'huiles végétales, en éliminant les sels. La solution de glycérol brut comprend en poids de 88,5% de glycérol, 5,1% d'eau, 5,1% de chlorure de sodium. Un flux de 8642g est envoyé en continu en alimentation d'un réacteur agité de 2 litres chauffé par un chauffe réacteur électrique extérieur. Les vapeurs de glycérol et eau sont condensées dans un réfrigérant et récupérées dans un récepteur. Cette opération de purification est réalisée sous une pression de 670 Pa. On obtient 7695g d'une solution de glycérol exempte de chlorure de sodium.

### Exemple 2

Dans une deuxième phase, on réalise la réaction de déshydratation du glycérol en acroléine et la condensation d'une partie de l'eau. La réaction de déshydratation est effectuée en phase gaz dans un réacteur en lit fixe en présence d'un catalyseur solide constitué par une zircone tungstée ZrO₂-WO₃ à une température de 320°C, sous pression atmosphérique. Un mélange de glycérol (20% massique) et eau (80% massique) est envoyé dans un vaporiseur, en présence d'air, dans un ratio molaire O₂ / glycérol de 0,6 / 1. Le milieu gazeux sortant du vaporiseur à 290°C est introduit dans le réacteur, constitué d'un tube de diamètre 30mm chargé de 400 ml de catalyseur, plongé dans un bain de sel (mélange eutectique KNO₃, NaNO₃, NaNO₂) maintenu à la température de 320°C. En sortie du réacteur, le mélange réactionnel gazeux est envoyé en pied d'une colonne de condensation. Cette colonne est constituée d'une section inférieure remplie d'anneaux Raschig, surmontée d'un condenseur dans lequel circule un fluide caloporteur froid. La température de refroidissement dans les échangeurs est adaptée de façon à obtenir en tête de colonne une température des vapeurs de 72°C sous pression atmosphérique. Dans ces conditions, la perte d'acroléine en pied de colonne de condensation est inférieure à 5%.

### Exemple 3

Dans une troisième phase, le mélange gazeux contenant 1,75mol/h acroléine est introduit, après addition d'air (ratio molaire O₂ / acroléine de 0,8 / 1) et d'azote en quantité nécessaire pour obtenir une concentration d'acroléine de 6,5% molaire, en alimentation du réacteur d'oxydation de l'acroléine en acide acrylique. Ce réacteur d'oxydation est constitué d'un tube de diamètre 30mm chargé de 480ml de catalyseur à base d'oxyde mixte MoN, plongé dans un bain de sel identique à celui décrit dans l'exemple 2, maintenu à une température de 250°C. Avant introduction sur le lit catalytique, le mélange gazeux est préchauffé dans un tube plongeant également dans le bain de sel.

### Exemple 4

La quatrième phase consiste à récupérer l'acide acrylique contenu dans le mélange gazeux sous forme d'une solution aqueuse (acide acrylique brut). En sortie de deuxième réacteur, le mélange gazeux est introduit en pied d'une colonne d'absorption à contre-courant avec de l'eau, fonctionnant sous pression atmosphérique. Cette colonne est remplie de garnissage vrac du type ProPak. En tête de cette colonne, on introduit 110g/h d'eau à température ambiante. En partie inférieure, sur 1/3 de sa hauteur totale, la colonne est équipée d'une section de refroidissement, qui reçoit en partie basse le gaz réactionnel introduit à 150°C. Une partie du liquide de pied de colonne est soutiré, refroidi à travers un échangeur externe, puis recyclé en tête de cette section inférieure de refroidissement. La température des vapeurs en tête de colonne est de 75°C, celle de la solution aqueuse d'acide acrylique brut obtenue en pied de colonne est de 80°C. Le produit obtenu en pied (acide acrylique brut) contient en poids 50% d'acide acrylique, 6,5% d'acide acétique, 43% d'eau.

### Exemple 5

La cinquième phase consiste à purifier l'acide acrylique brut pour obtenir le grade acide acrylique technique. Pour ce faire, on utilise une série de distillations successives. La solution aqueuse obtenue est soumise d'abord à une distillation pour éliminer l'eau sous forme d'un mélange azéotropique avec la méthylisobutylcétone (MIBK). La colonne, garnie d'éléments ProPak représentant une efficacité de 15 plateaux théoriques, est alimentée en son milieu par l'acide acrylique brut, et en tête par la MIBK, dans un ratio massique MIBK / eau contenue dans l'acide acrylique brut de 3 / 1. Le mélange azéotropique distille à une température de tête de 45°C sous une pression de 1,2.10⁴ Pa. L'acide acrylique déshydraté récupéré en pied de colonne ne contient plus que 1% d'eau en poids. Il est envoyé en alimentation d'une colonne d'étêtage, qui permet d'éliminer en tête, les composés légers, essentiellement l'acide acétique. Cette colonne, garnie d'éléments ProPak (20 plateaux théoriques), est alimentée en son milieu par le flux d'acide acrylique déshydraté, et on distille en tête un flux « riche » en acide acétique sous une pression de 5,3.10³ Pa, à une température de tête de 38°C.
L'acide acrylique étêté récupéré en pied de cette colonne titre 0,1 % poids d'acide acétique. Il est envoyé en alimentation d'une colonne d'équeutage garnie de 17 plateaux perforés à déversoirs, qui permet d'éliminer les composés lourds en pied. Cette colonne fonctionne sous une pression de 1,2.10⁴ Pa, avec une température de tête de 81°C, avec un taux de reflux de 1. L'acide acrylique obtenu en tête de colonne constitue l'acide acrylique technique (AAT).
Les analyses de l'acide acrylique grade technique montrent que le produit contient en poids 0,0005 % de protoanémonine, 0,0032% de furfural, 0,0005 % de benzaldéhyde, 0,005% d'acroléine, 0,11 % d'acide acétique, 0,0019 % d'anhydride maléique.

### Exemple 6

L'acide acrylique de grade technique est additionné d'hydrate d'hydrazine dans un ratio molaire de 7/1 par rapport aux aldéhydes présents (furfural, benzaldéhyde, acroléine ...) et le flux est distillé dans une colonne de 17 plateaux perforés à déversoirs, sous une pression de 9.10³ Pa, avec une température de tête de 69°C.
Dans toutes les étapes de distillations précédemment décrites, on introduit des inhibiteurs de polymérisation en tête des colonnes, de façon à éviter la formation de polymères. Les inhibiteurs utilisés sont la phénothiazine (PTZ), l'hydroquinone et l'éther méthylique de l'hydroquinone (EMHQ) à des teneurs classiques connues de l'homme du métier.
Dans la dernière colonne de distillation, la phénothiazine est introduite au niveau du plateau n°5 compté depuis la tête, et on introduit de l'EMHQ en tête, en solution dans l'acide acrylique grade polymère On introduit enfin un complément d'EMHQ de façon à obtenir une concentration de 0,02% de cet inhibiteur de polymérisation dans l'acide acrylique de grade polymère.
Les analyses de l'acide acrylique obtenu, de grade polymère, montrent que le produit contient 1,5 ppm de protoanémonine, 0,8 ppm de furfural, 0,5 ppm de benzaldéhyde, 0,2 ppm d'acroléine , 4 ppm d'acétaldéhyde, 0,6 ppm de formaldéhyde, 0,15% d'acide acétique, 12 ppm d'anhydride maléique, 0,2 ppm de PTZ. Le produit obtenu est exempt d'hydrazine.
Par ailleurs, la teneur en ¹⁴C de l'échantillon, mesurée selon la norme ASTM D6866-06, selon la méthode par spectrométrie de masse sera telle que le ratio ¹⁴C /¹²C sera supérieur à 0,9.10⁻¹².

### Exemple 7 (comparatif)

La solution aqueuse d'acide acrylique (acide acrylique brut) obtenue dans l'exemple 4 (540g) est mélangée comme dans l'exemple 2 de la demande WO2006/092272 avec 50% de son poids de toluène. Ce mélange est placé sous agitation vigoureuse dans un récipient à double-enveloppe refroidi à 0°C, puis l'agitation est arrêtée et on laisse décanter les 2 phases non miscibles pendant une heure. La phase organique riche en toluène (61,%) est la phase supérieure. Cette phase organique (307g) est séparée. Elle contient 30% AA, soit seulement 34,7% de l'AA contenu dans le mélange initial. Cette solution est distillée dans une colonne, garnie d'éléments ProPak représentant une efficacité de 5 plateaux théoriques. Une première fraction de 226g correspondant au mélange azéotropique riche en toluène et eau, distillant à une température de tête comprise entre 31 et 43°C sous une pression de 9.1.10³ Pa, est recueillie. Elle contient 18,6g AA (soit une perte de 6,9% de l'AA contenu dans la solution initiale). Une deuxième fraction correspondant au produit pur distille à une température de tête de 76°C. Elle titre 97,6% AA et 0,11% d'eau. Ce produit purifié, soit 52 g, représente seulement 19,3% de l'AA contenu dans la solution initiale, soit un rendement de récupération beaucoup trop faible pour que le procédé soit économiquement acceptable. En outre, l'analyse de ce produit fait apparaître des concentrations en poids d'impuretés qui le rendent impropre à une application en vue de la production de polymères : 1,47% d'acide acétique, 75 ppm de furfural, 41 ppm de benzaldéhyde, 17 ppm de protoanémonine, 8 ppm d'anhydride maléique.

## Revendications

1. Procédé de fabrication d'un acide acrylique bio-ressourcé de grade polymère ayant une teneur poids en acide acrylique > 99 %, et des teneurs en impuretés suivantes protoanémomine < 5 ppm, de préférence < 3 ppm, aldéhydes totaux < 10 ppm, de préférence < 3 ppm, anhydride maléique < 30 ppm, de préférence < 15 ppm, inhibiteurs de polymérisation non phénoliques < 10 ppm, de préférence < 3 ppm, et une teneur en masse de ¹⁴C tel que le ratio ¹⁴C /¹²C > 0,8.10⁻¹², de préférence > 1.10⁻¹²,
à partir de glycérol **caractérisé en ce qu'**il comporte les étapes suivantes :
i) déshydratation du glycérol en acroléine,
ii) oxydation de l'acroléine formée en acide acrylique,
iii) extraction de l'acide acrylique formé par absorption à contre-courant sous forme d'une solution aqueuse d'acide acrylique,
iv) déshydratation de la solution en présence d'un solvant de l'acide acrylique non miscible à l'eau mais susceptible de former avec l'eau un azéotrope,
v) élimination des composés légers, en particulier l'acide acétique et l'acide formique, par distillation,
vi) élimination des impuretés lourdes par distillation, pour obtenir une qualité d'acide acrylique de grade "technique",
vii) purification par distillation de l'acide acrylique technique pour obtenir une qualité d'acide acrylique de grade "polymère", après addition à l'acide acrylique d'un composé aminé réagissant avec les aldéhydes encore présents, choisi parmi les dérivés de l'hydrazine, de préférence l'hydrate d'hydrazine.

2. Procédé selon la revendication 1 **caractérisé en ce que** l'étape iii) met en oeuvre l'eau comme solvant d'absorption.

3. Procédé selon la revendication 1 ou 2 **caractérisé en ce que** lors de l'étape iii) la colonne d'absorption est couplée avec une colonne de distillation des composés très légers tels que l'acroléine non convertie.

4. Procédé selon l'une quelconque des revendications précédentes **caractérisé en ce que** l'étape iv) mise en oeuvre avec un solvant est réalisée par une extraction liquide-liquide, suivie d'une séparation de l'acide acrylique contenue dans la phase organique par distillation.

5. Procédé selon l'une quelconque des revendications 1 à 3 **caractérisé en ce que** l'étape iv) mise en oeuvre avec un solvant est réalisée par distillation azéotropique.

6. Procédé selon l'une quelconque des revendications précédentes **caractérisé en ce que** l'étape iv) mise en oeuvre avec un solvant est couplée à une deuxième colonne de récupération du solvant.

7. Procédé selon l'une quelconque des revendications 4 à 6 **caractérisé en ce que** le solvant est la MIBK.

8. Procédé selon l'une quelconque des revendications précédentes **caractérisé en ce que** l'étape vii) de purification de l'acide acrylique technique est mise en oeuvre à l'aide de deux colonnes de distillation.

9. Procédé selon l'une quelconque des revendications précédentes **caractérisé en ce que** le composé aminé est.
introduit dans un ratio molaire de 2 à 10 par rapport aux aldéhydes contenus dans l'acide acrylique de grade technique.

## Patentansprüche

1. Verfahren zur Herstellung von aus biologischen Quellen stammender Acrylsäure von Polymer-Qualität mit einem Acrylsäuregehalt > 99 Gew.-% und Gehalten der folgenden Vereinigungen: Photo-anemonin < 5 ppm, vorzugsweise < 3 ppm, Aldehyden insgesamt < 10 ppm, vorzugsweise < 3 ppm, Maleinsäureanhydrid < 30 ppm, vorzugsweise < 15 ppm, nichtphenolischen Polymerisationsinhibitoren < 10 ppm, vorzugsweise < 3 ppm, und einem solchen ¹⁴C-Gewichtsgehalt, dass das ¹⁴C/¹²C-Verhältnis > 0,8.10⁻¹², vorzugsweise > 1.10⁻¹² ist,
aus Glycerin, **dadurch gekennzeichnet, dass** es die folgenden Schritte umfasst:
i) Dehydratisierung von Glycerin zu Acrolein,
ii) Oxidation von Acrolein zu Acrylsäure,
iii) Extraktion der gebildeten Acrylsäure durch Gegenstromabsorption in Form einer einer wässrigen Acrylsäurelösung,
iv) Dehydratisierung der Lösung in Gegenwart eines Lösungsmitteln für Acrylsäure, das mit Wasser nicht mischbar ist, aber mit Wasser ein Azeotrop bilden kann,
v) Entfernung der leichten Verbindungen, insbesondere Essigsäure und Ameisensäure, durch Destillation,
vi) Entfernung der schweren Verunreinigungen durch Destillation zum Erhalt einer Acrylsäure "technischer" Qualität,
vii) Reinigung der technischen Acrylsäure durch Destillation zum Erhalt einer Acrylsäure von "Polymer"-Qualität nach Zugabe einer Aminverbindung, die mit den noch vorhandenen Aldehyden reagiert und aus Hydrazinderivaten, vorzugsweise Hydrazinhydrat, ausgewählt wird, zu der Acrylsäure.

2. Verfahren nach Anspruch 1, **dadurch gekennzeichnet, dass** in Schritt iii) Wasser als Adsorptionslösungsmittel eingesetzt wird.

3. Verfahren nach Anspruch 1 oder 2, **dadurch gekennzeichnet, dass** in Schritt iii) die Absorptionskolonne mit einer Kolonne zur Destillation der sehr leichten Verbindungen, wie nicht umgesetztem Acrolein, gekoppelt wird.

4. Verfahren nach einem der vorhergehenden Ansprüche, **dadurch gekennzeichnet, dass** der mit einem Lösungsmittel durchgeführte Schritt iv) durch eine Flüssig-Flüssig-Extraktion gefolgt von einer Abtrennung der in der organischen Phase enthaltenen Acrylsäure durch Destillation durchgeführt wird.

5. Verfahren nach einem der Ansprüche 1 bis 3, **dadurch gekennzeichnet, dass** der mit einem Lösungsmittel durchgeführte Schritt iv) durch azeotrope Destillation durchgeführt wird.

6. Verfahren nach einem der vorhergehenden Ansprüche, **dadurch gekennzeichnet, dass** der mit einem Lösungsmittel durchgeführte Schritt iv) mit einer zweiten Kolonne zur Rückgewinnung des Lösungmittels gekoppelt wird.

7. Verfahren nach einem der Ansprüche 4 bis 6, **dadurch gekennzeichnet, dass** es sich bei dem Lösungsmittel um MIBK handelt.

8. Verfahren nach einem der vorhergehenden Ansprüche, **dadurch gekennzeichnet, dass** der Schritt vii) der Reinigung der technischen Acrylsäure mit Hilfe von zwei Destillationskolonnen durchgeführt wird.

9. Verfahren nach einem der vorhergehenden Ansprüche, **dadurch gekennzeichnet, dass** die Aminoverbindung in einem Molverhältnis von 2 bis 10, bezogen auf die in der Acrylsäure technischer Qualität enthaltenden Aldehyde, eingetragen wird.

## Claims

1. Process for the manufacture of bioresourced acrylic acid of polymer grade having a content by weight of acrylic acid > 99% and contents of the following impurities: protoanemonin < 5 ppm, preferably < 3 ppm, total aldehydes < 10 ppm, preferably < 3 ppm, maleic anhydride < 30 ppm, preferably < 15 ppm, nonphenolic polymerization inhibitors < 10 ppm, preferably < 3 ppm, and a content by weight of ¹⁴C such that the ¹⁴C/¹²C (ratio > 0,8 × 10⁻¹² preferably > 1 × 10⁻¹², from glycérol, **characterized in that** it comprises the following stages:
i) dehydration of the glycerol to give acrolein,
ii) oxidation of the acrolein formed to give acrylic acid,
iii) extraction of the acrylic acid formed by counter-currentwise absorption in the form of an aqueous acrylic acid solution,
iv) dehydration of the solution in the presence of a solvent for acrylic acid which is immiscible with water but capable of forming an azeotrope with water,
v) removal of the light compounds, in particular acetic acid and formic acid, by distillation
vi) removal of the heavy impurities by distillation, in order to obtain an acrylic acid of "technical" grade,
vii) purification by distillation of the technical acrylic acid, in order to obtain an acrylic acid of "polymer" grade, after addition to the acrylic acid of an amino compound which reacts with the aldehydes still present, chosen from hydrazine derivatives, preferably hydrazine hydrate.

2. Process according to Claim 1, **characterized in that** stage iii) employs water as absorption solvent.

3. Process according to Claim 1 or 2, **characterized in that**, during stage iii), the absorption column is coupled with a column for the distillation of the very light compounds, such as unconverted acrolein.

4. Process according to any one of the preceding claims, **characterized in that** stage iv), employed with a solvent, is carried out by a liquid/liquid extraction, followed by a separation, by distillation, of the acrylic acid present in the organic phase.

5. Process according to any one of Claims 1 to 3, **characterized in that** stage iv), employed with a solvent, is carried out by azeotropic distillation.

6. Process according to any one of the preceding claims, **characterized in that** stage iv), employed with a solvent, is coupled to a second column for recovery of the solvent.

7. Process according to any one of Claims 4 to 6, **characterized in that** the solvent is MIBK.

8. Process according to any one of the preceding claims, **characterized in that** stage vii) of purification of the technical acrylic acid is carried out using two distillation columns.

9. Process according to any one of the preceding claims, **characterized in that** the amino compound is introduced in a molar ratio of 2 to 10 with respect to the aldehydes present in the acrylic acid of technical grade.
